Europäisches Patentamt

(19) European Patent Office

Office européen des brevets

(11) Publication number: **0 000 144**
**B1**

# EUROPEAN PATENT SPECIFICATION

(45) Date of publication of patent specification: **05.08.81**

(21) Application number: **78100156.5**

(22) Date of filing: **14.06.78**

(51) Int. Cl.³: **C 07 C 118/00,**
**C 07 C 119/042**

(54) **Process for preparing 2-isocyanatoalkyl esters of unsaturated carboxylic acids.**

(30) Priority: **15.06.77 US 806805**

(43) Date of publication of application:
**10.01.79 Bulletin 79/1**

(45) Publication of the grant of the European patent:
**05.08.81 Bulletin 81/31**

(84) Designated Contracting States:
**DE FR GB NL**

(56) References cited:
**DE - A - 1 913 273**
**FR - A - 1 217 276**
**US - A - 2 821 544**

(73) Proprietor: **THE DOW CHEMICAL COMPANY**
**2030 Abbott Road Post Office Box 1967**
**Midland, Michigan 48640 (US)**

(72) Inventor: **Burdett, Kenneth Allen**
**2800 Dina**
**Midland, Michigan (US)**

(74) Representative: **Weickmann, Heinrich, Dipl.-Ing.**
**et al,**
**Postfach 860 820 Möhlstrasse 22**
**D-8000 München 86 (DE)**

Courier Press, Leamington Spa, England.

Process for preparing 2-isocyanatoalkyl esters of unsaturated carboxylic acids.

This invention is directed to a process for preparing a 2-isocyanatoalkyl ester of an unsaturated carboxylic acid by reacting a water-soluble 2-alkenyl-2-oxazoline with a solution of phosgene in a water-immiscible organic solvent in the presence of an aqueous solution of a hydrochloric acid acceptor characterized by the addition of the 2 - alkenyl - 2 - oxazoline into the reaction mixture as an aqueous solution, the aqueous solution of the 2-alkenyl-2-oxazoline being prepared by (A) reacting a 2-alkyl-2-oxazoline with formaldehyde to form 2-($\alpha$-hydroxymethylalkyl)-2-oxazoline, (B) dehydrating the 2 - ($\alpha$ - hydroxy - methylalkyl)-2-oxazoline to form the 2 - alkenyl - 2 - oxazoline, and (C) separating a volatile composition comprising water and 2 - alkenyl - 2 - oxazoline which condenses to an aqueous solution of the 2 - alkenyl - 2 - oxazoline.

The present process is a substantial advance over the closest known art, British Patent 1,252,099, which requires that the 2 - alkenyl - 2 - oxazoline be introduced into the reaction as a solution in a water-immiscible solvent such as methylene chloride. It is now no longer necessary to prepare anhydrous 2 - alkenyl - 2 - oxazolines to be dissolved in water - immiscible solvents. The total volume of water-immiscible solvents used in the process is substantially reduced over the process of the art, which results in further economy. There is also a significant advantage in terms of occupational safety. The 2 - alkenyl - 2 - oxazolines, particularly the lower molecular weight compounds such as 2 - vinyl - 2 - oxazoline and 2 - isopropenyl - 2 - oxazoline, are treated as a toxic class of compounds. The potential exposure by inhalation is reduced when aqueous solutions of 2 - alkenyl - 2 - oxazolines are employed in comparison with employing solutions in water-immiscible solutions. These improvements of the present process result in economic and safety advantages over the prior art which are commercially significant.

Prior art methods of preparing 2-isocyanatoalkyl esters of unsaturated carboxylic acids have utilized 2 - alkenyl - 2 - oxazolines prepared from expensive reagents in multi-step processes. Product yields were often low. See Angew Chem. Volume 78, pages 913 and following, published in 1966.

The process of the present invention requires an aqueous solution of 2 - alkenyl - 2 - oxazolines prepared by (A) reacting a 2 - alkyl - 2 - oxazoline with formaldehyde to form a 2 - ($\alpha$ - hydroxymethylalkyl) - 2 - oxazoline, (B) dehydrating the 2 - ($\alpha$ - hydroxymethylalkyl) - 2 - oxazoline to form the 2 - alkenyl - 2 - oxazoline, and (C) separating a volatile composition comprising water and 2 - alkenyl - 2 - oxazoline which condenses to an aqueous solution of the 2 - alkenyl - 2 - oxazoline. The preferred process for preparing the aqueous solution of the 2 - alkenyl - 2 - oxazoline is described in DE-OS 27 27 824.

Suitable 2 - alkyl - 2 - oxazolines are those oxazolines in which the 2-alkyl group contains from 1 to 3 carbon atoms. The oxazoline ring may optionally contain alkyl groups, in the 4- and/or 5-ring positions as long as the resultant 2 - alkenyl - 2 - oxazolines are water-soluble. The most preferred 2 - alkyl - 2 - oxazolines are 2 - methyl - 2 - oxazoline and 2 - ethyl - 2 - oxazoline.

The yield of the desired 2 - ($\alpha$ - hydroxymethylalkyl) - 2 - oxazoline is maximized when the molar ratio of oxazoline to formaldehyde is greater than 1:1. Normally, at least 1.5 moles of 2 - alkyl - 2 - oxazoline per mole of formaldehyde is employed. The preferred ratio of reactants is from 2 to 10 moles of oxazoline per mole of formaldehyde. The most preferred ratio is 3 to 5 moles of oxazoline per mole of formaldehyde.

Product yields of the 2 - ($\alpha$ - hydroxymethylalkyl) - 2 - oxazoline are also maximized by conducting step A under anhydrous or substantially anhydrous conditions. The oxazoline reactant is preferably predried, employing such drying agents as, for example, molecular sieves or solid sodium hydroxide. Paraformaldehyde having a 95 percent or greater formaldehyde content is the preferred formaldehyde source.

Step A is conducted at any suitable temperature that promotes the reaction and is below the decomposition temperature of the desired product. Satisfactory reaction rates have been observed at temperatures of from 90°C to 115°C. Temperatures of from 95°C to 105°C are preferred. At those temperatures, reaction times of from 2 to 8 hours are conventional. Inert organic solvents such as, for example, benzene or toluene may be employed if desired. Preferably the process is conducted without employing a solvent.

The 2 - ($\alpha$ - hydroxymethylalkyl) - 2 - oxazoline is recovered from the reaction product of step A by conventional techniques. Fractional distillation under reduced pressure at a temperature below the decomposition temperature of the 2 - ($\alpha$ - hydroxymethylalkyl) - 2 - oxazoline is preferred. The excess 2 - alkyl - 2 - oxazoline and water co-distill first and are recovered. The 2 - ($\alpha$ - hydroxymethylalkyl) - 2 - oxazolines are higher boiling. They are preferably further purified by such conventional techniques as, for example, distillation employing a falling film still.

The 2 - ($\alpha$ - hydroxymethylalkyl) - 2 - oxazolines are dehydrated to form the 2-alkenyl-2-oxazoline by contacting the reactant with an alkali or alkaline earth metal hydroxide. The dehydration reaction is conducted at a temperature of from 95°C to 200°C under reduced pressure such as, for example, 10 to 150 mm of mercury.

The efficiency of the alkali or alkaline earth metal hydroxide as a dehydration catalyst tends to correlate with the solubility of the hydroxide in hot water. The more soluble hydroxides are the more efficient catalysts. The preferred catalysts are lithium hydroxide, sodium hydroxide, potassium hydroxide, and barium hydroxide. Most preferred is sodium hydroxide.

The dehydration step may be conducted batchwise or continuously, the continuous process being preferred. In the continuous process, the 2 - ($\alpha$ - hydroxymethylalkyl) - 2 - oxazoline is added to the dehydration catalyst at the desired reaction temperature. The 2 - alkenyl - 2 - oxazoline product is volatilized at the reaction temperature under reduced pressure and co-distills with water from the reaction vessel. Preferably, the 2 - ($\alpha$ - hydroxymethylalkyl) - 2 - oxazoline is metered into the reaction vessel at substantially the same rate at which the 2 - alkenyl - 2 - oxazoline/water mixture is removed as overheads. When cooled to room temperature, the product is a solution of water and 2 - alkenyl - 2 - oxazoline.

Inert solvents which are liquid at the reaction temperature may be employed in the dehydration step. Lower alkyl monoethers of polyalkylene glycols are solvents for alkali and alkaline earth metal hydroxides and are preferred solvents for this step. Suitable compounds include, for example, the methyl, ethyl, propyl and butyl ethers of diethylene glycol and triethylene glycol. The preferred solvent is the monomethyl ether of triethylene glycol when sodium hydroxide is employed as the catalyst.

The crude aqueous solution of 2 - alkenyl - 2 - oxazoline is surprisingly useful in the present process. The aqueous solution of the 2 - alkenyl - 2 - oxazoline can be added per se into the reaction mixture or it can be further diluted with water before adding it to the reaction mixture. It is important that there be sufficient water present in the reaction mixture to create two phases with the water-immiscible solvent. The 2 - alkenyl - 2 - oxazoline is an effective coupling agent. An insufficient amount of water in the reaction mixture would result in a single phase, which is not desirable. Preferably at least 15 moles of water per mole of oxazoline reactant is employed in the reaction mixture. Most preferably the proportion of water is at least 25 moles of water per mole of oxazoline reactant.

Phosgene is employed as a solution in an inert water-immiscible organic solvent. Examples of suitable solvents include hydrocarbons such as hexane, cyclohexane, petroleum ether, benzene, toluene, xylene, and diisopropylbenzene; and chlorinated hydrocarbons such as methylene chloride, chloroform, chlorobenzene, and ortho-dichlorobenzene. Mixtures of such solvents may also be employed. Methylene chloride is the preferred solvent.

Suitable hydrochloric acid acceptors include both inorganic and organic bases such as, for example, sodium and potassium hydroxides, sodium and potassium carbonates, sodium and potassium phosphates, triethylamine and pyridine. The inorganic water-soluble bases are preferred due to cost and ease of handling. Sodium hydroxide is the most preferred acid acceptor.

The reaction step to produce the 2 - isocyanatoalkyl ester is normally conducted at a temperature of from −30°C to 25°C, preferably from −10°C to 15°C, and more preferably from 0°C to 10°C. This reaction step is preferably conducted by simultaneously introducing a pre-cooled aqueous solution of the 2 - alkenyl - 2 - oxazoline, a pre-cooled organic solution of phosgene and a pre-cooled aqueous solution of the hydrochloric acid acceptor into a reaction vessel with vigorous stirring and cooling. The reaction is essentially instantaneous and is normally complete upon thorough mixing of the reactants. This step can be conducted batchwise or in a continuous fashion.

The 2 - isocyanatoalkyl ester of the unsaturated carboxylic acid is recovered from the organic phase of the reaction mixture by conventional techniques such as, for example, distillation. Product yields are maximized by recovering the product from the organic phase as soon as practical to minimize losses due to hydrolysis. Examples 1A to 1B are illustrative of the feasibility of the individual steps making up the process of claim 1.

### Example 1A
Preparation of 2 - Isopropenyl - 2 - oxazoline

2 - Ethyl - 2 - oxazoline (594 g; 6.0 moles) and 95 percent paraformaldehyde (63.2 g; 2.0 moles) were charged to a reaction vessel equipped with a mechanical stirrer and condenser. The reaction mixture was heated to 100°C with stirring and maintained under these conditions for 4 hours. A sample of the reaction mixture was then analyzed by vapor phase chromatography with the following results: 60.7 weight percent 2 - ethyl - 2 - oxazoline; 37.9 weight percent 2 - ($\alpha$ - hydroxy - methylethyl) - 2 - oxazoline; and the remaining 1.4 weight percent was not identified. On these data, the conversion of 2 - ethyl - 2 - oxazoline was 98.5 percent and the percent yield of 2 - ($\alpha$ - hydroxymethylethyl) - 2 - oxazoline was 96.5 percent. The excess 2 - ethyl - 2 - oxazoline was removed from the reaction mixture by distillation under reduced pressure leaving the desired 2 - ($\alpha$ - hydroxymethylethyl) - 2 - oxazoline as the still bottoms.

Sodium hydroxide beads (60.0 g; 1.5 mole) were added to a reaction vessel equipped with a mechanical stirrer, a dropping funnel and a distillation column packed with 1/4 inch (0.64 cm) glass beads. This material was heated to a pot temperature of approximately 175°C at a pressure of 150 mm Hg. To this heated system was added the 2 - ($\alpha$ - hydroxymethylethyl) - 2 - oxazoline from the above (containing 100 ppm of a polymerization inhibitor) at a rate of approximately 1 g per minute. All volatiles passing through the distillation column were collected in a cold trap and analyzed by vapor phase chromatography using 1,2,4-trichlorobenzene as an internal standard. The mixture contained 2.5 weight percent unreacted 2-ethyl-2-oxazoline; 11.7 weight percent water; and 85.8 weight percent 2 - isopropenyl - 2 - oxazoline. This amounts to a 97.8 percent yield of 2 - isopropenyl - 2 - oxazoline.

Similar high yields were obtained when the dehydration was conducted using sodium hydroxide dissolved in monomethyl ether of triethylene glycol and a minor amount of water. Data obtained on a series of such dehydrations indicate that the effective life of the sodium hydroxide catalyst was extended by using this material as a reaction medium.

Example 1B
Preparation of 2-Isocyanatoethyl Methacrylate

A 3-liter jacketed reactor vessel was charged with 100 ml of methylene chloride and cooled to approximately 0°C. A solution of 2 - isopropenyl - 2 - oxazoline (100 g) in 177 ml of water, a solution of phosgene (131.5 g) in 400 ml of methylene chloride, and 250 ml of a solution of 35 weight percent sodium hydroxide in water were added simultaneously to the reaction vessel with stirring and cooling. The rates of addition were such that the three reagents were added over approximately a 50 minute time span with the temperature being maintained at 10°C to 18°C. Stirring was continued for two minutes and the layers allowed to separate. The organic layer was washed twice with 100 ml portions of a saturated aqueous sodium bicarbonate solution, dried over sodium sulfate and concentrated under reduced pressure. The colorless concentrate was inhibited with 0.1 g of phenothiazine and the desired product recovered therefrom as a colorless liquid (133.6 g) boiling at 46—47°C/0.4 mm Hg. Product yield 95.7 percent of theory.

**Claims**

1. A process for preparing a 2 - isocyanato - alkyl ester of an unsaturated carboxylic acid by reacting a water-soluble 2 - alkenyl - 2 - oxazoline with a solution of phosgene in a water-immiscible organic solvent in the presence of an aqueous solution of a hydrochloric acid acceptor and sufficient water to create two phases with the water-immiscible solvent characterized by adding the 2 - alkenyl - 2 - oxazoline into the reaction mixture as an aqueous solution, the aqueous solution of the 2 - alkenyl - 2 - oxazoline being prepared by

(a) reacting a 2 - alkyl - 2 - oxazoline with formaldehyde to form 2 - $\alpha$ - hydroxy-methylalkyl) - 2 - oxazoline,

(b) dehydrating the 2 - ($\alpha$ - hydroxy - methylalkyl) - 2 - oxazoline to form the 2 - alkenyl - 2 - oxazoline by contacting the reactant with an alkali or alkaline earth metal hydroxide at a temperature of from 95°C to 200°C under reduced pressure, and

(c) separating a volatile composition from the reaction product at (b) which is condensed to give the aqueous solution of the 2 - alkenyl - 2 - oxazoline.

2. The process of claim 1, characterized in that the 2-alkyl group of the 2 - alkyl - 2 - oxazoline compound contains 1 to 3 carbon atoms.

**Revendications**

1. Procédé pour la préparation d'un ester 2-isocyanatoalkylique d'un acide carboxylique insaturé par réaction d'une 2 - alcényl - 2 - oxazoline soluble dans l'eau avec une solution de phosgène dans un solvant organique non-miscible avec l'eau, en présence d'une solution aqueuse d'un accepteur d'acide chlorhydrique et d'une quantité suffisante d'eau pour créer deux phases avec le solvant non-miscible avec l'eau, caractérisé par l'addition de la 2 - alcényl - 2 - oxazoline dans le mélange réactionnel sous forme d'une solution aqueuse, la solution aqueuse de la 2 - alcényl - 2 - oxazoline étant préparée par:

(a) la réaction d'une 2 - alkyl - 2 - oxazoline avec le formaldéhyde pour former une 2 - ($\alpha$ - hydroxyméthyl - alkyl) - 2 - oxazoline,

(b) la déshydratation de la 2 - ($\alpha$-hydroxyméthylalkyl) - 2 - oxazoline pour former la 2 - alcényl - 2 - oxazoline en mettant en contact le réactif avec un hydroxyde de métal alcalin ou de métal alcalino-terreux, à une température allant de 95° à 200°C, sous pression réduite, et

(c) la séparation d'une composition volatile d'avec le produit de réaction pendant (b), qui est condensée pour donner la solution aqueuse de la 2 - alcényl - 2 - oxazoline.

2. Procédé selon la revendication 1, caractérisé par le fait que le groupe 2 - alkyle du composé 2 - alkyl - 2 - oxazoline contient 2 à 3 atomes de carbone.

**Patentansprüche**

1. Verfahren zur Herstellung eines 2 - Isocyanatoalkylesters einer ungesättigten Carbonsäure durch Umsetzung eines wasserlöslichen 2 - Alkenyl - 2 - oxazolins mit einer Lösung von Phosgen in einem mit Wasser unmischbaren organischen Lösungsmittel in Gegenwart einer wässrigen Lösung eines Chlorwasserstoffsäureakzeptors und ausreichend

Wasser um mit dem mit Wasser unmischbaren Lösungsmittel zwei Phasen zu bilden, dadurch gekennzeichnet, daß das 2 - Alkenyl - 2 - oxazolin der Reaktionsmischung in Form einer wässrigen Lösung zugesetzt wird, wobei diese wässrige Lösung von 2 - Alkenyl - 2 - oxazolin hergestellt wird durch

(a) Umsetzung eines 2 - Alkyl - 2 - oxazolins mit Formaldehyd unter Bildung von 2 - ($\alpha$ - Hydroxymethyl - alkyl) - 2 - oxazolin,

(b) Dehydratisieren des 2 - ($\alpha$ - Hydroxy - methylalkyl) - 2 - oxazolins unter Bildung des 2 - Alkenyl - 2 - oxazolins durch Kontaktieren des Reaktanten mit einem Alkali- oder Erdalkalimetallhydroxid bei einer Temperatur von 95°C bis 200°C unter vermindertem Druck und

(c) Abtrennung einer flüchtigen Zusammensetzung vom Reaktionsprodukt in Stufe b, welche zu der wässrigen Lösung des 2 - Alkenyl - 2 - oxazolins kondensiert wird.

2. Verfahren nach Anspruch 1, dadurch gekennzeichnet, daß die 2 - Alkylgruppe der 2 - Alkyl - 2 - oxazolin - Verbindung 1 bis 3 Kohlenstoffatome enthält.